# EUROPÄISCHE PATENTSCHRIFT

(11) **EP 0 826 775 B1**
(45) Veröffentlichungstag und Bekanntmachung des Hinweises auf die Patenterteilung: **23.04.2003**
(21) Anmeldenummer: 97114569.3
(22) Anmeldetag: 22.08.1997
(51) Int. Cl.: C12N 15/63, C12N 15/62, C12N 15/85, C12N 15/86, C12N 7/01, C12N 5/10, A61K 31/70, C07K 14/82, C12N 15/53, C12N 15/54

(54) **Vektoren zur konditionalen Genexpression**
Vector for conditional gene expression
Vecteur pour l'expression conditionnelle

(30) Priorität: 02.09.1996 DE 19635568
(43) Veröffentlichungstag der Anmeldung: 04.03.1998
(73) Patentinhaber: GSF-Forschungszentrum für Umwelt und Gesundheit GmbH, 85764 Oberschleissheim (DE)
(72) Erfinder: Polack, Axel, 80798 München (DE); Christoph, Barbara, 80798 München (DE); Kempkes, Bettina, Chestnut Hill, MA 02167 (US)
(74) Vertreter: Reinhard - Skuhra - Weise & Partner

(56) Entgegenhaltungen:
- WO-A-93/04179
- WO-A-94/04672
- WO-A-94/12629
- WO-A-94/29442
- WO-A-96/01313
- WO-A-96/40892
- WO-A-96/40946
- WO-A-97/35992
- LIANG X ET AL: "NOVEL, HIGH EXPRESSING AND ANTIBIOTIC-CONTROLLED PLASMID VECTORS DESIGNED FOR USE IN GENE THERAPY" GENE THERAPY, Bd. 3, April 1996, Seiten 350-356, XP002037390
- GOSSEN M ET AL: "TIGHT CONTROL OF GENE EXPRESSION IN MAMMALIAN CELLS BY TETRACYCLINE-RESPONSIVE PROMOTERS" PROCEEDINGS OF THE NATIONAL ACADEMY OF SCIENCES OF USA, Bd. 89, Nr. 12, 15.Juni 1992, Seiten 5547-5551, XP000564458
- SHOCKETT P ET AL: "A MODIFIED TETRACYCLINE-REGULATED SYSTEM PROVIDES AUTOREGULATORY, INDUCIBLE GENE EXPRESSION IN CULTURED CELLS AND TRANSGENIC MICE" PROCEEDINGS OF THE NATIONAL ACADEMY OF SCIENCES OF USA, Bd. 92, Juli 1995, Seiten 6522-6526, XP002048532
- KIM H -J ET AL: "TETRACYCLINE REPRESSOR-REGULATED GENE REPRESSION IN RECOMBINANT HUMAN CYTOMEGALOVIRUS" JOURNAL OF VIROLOGY, Bd. 69, Nr. 4, April 1995, Seiten 2565-2573, XP002037393
- A.L. KIRCHMAIER AND B. SUGDEN: "Plasmid maintance of derivatives of oriP of Epstein-Barr virus" J. VIROLOGY, Bd. 69, Nr. 2, Februar 1995, AM.SOC.MICROBIOL.,WASHINGTON,US, Seiten 1280-1283, XP002048533
- W. HAMMERSCHMIDT AND B. SUGDEN: "Identification and characterization of oriLyt, a lytic origin of DNA replication" CELL, Bd. 55, 4.November 1988, CELL PRESS,CAMBRIDGE,MA,US;, Seiten 427-433, XP002048534
- C. MEITINGER ET AL.: "Crucial sequences within the Epstein-Barr virus TP1 promoter for EBNA2-mediated transactivation and interaction of EBNA2 with its responsive element" J. VIROLOGY, Bd. 68, Nr. 11, November 1994, AM.SOC.MICROBIOL.,WASHINGTON,US, Seiten 7497-7506, XP002048535
- HOSHIMARU M ET AL: "DIFFERENTIATION OF THE IMMORTALIZED ADULT NEURONAL PROGENITOR CELL LINE HC2S2 INTO NEURONES BY REGULATABLE SUPPRESSION OF THE V-MYC ONCOGENE" PROCEEDINGS OF THE NATIONAL ACADEMY OF SCIENCES OF USA, Bd. 93, Nr. 4, 20.Februar 1996, Seiten 1518-1523, XP002033326

## Beschreibung

Die vorliegende Erfindung betrifft in Eukaryontenzellen episomal replizierende Vektoren zur konditionalen Genex-pression in Eukaryonten, Verwendungen dieser Vektoren sowie Verfahren zur konditionalen Expression von Genen von Interesse unter Verwendung eines in Eukaryontenzellen episomal replizierenden Vektors.

Um die Funktion eines Genes zu studieren, werden Methoden benötigt, die eine gezielte An- und Abschaltung der Expression dieses Genes entweder in Zellkulturzellen oder im Versuchstier erlauben. Bisher bekannte Ansätze zur Lösung dieses Problems sehen vor: (i) temperatursensitive Mutanten des Gens herzustellen, die nur bei einer definierten Temperatur die gewünschte Funktion zeigen, (ii) die Verwendung von Promotoren, die durch Schwermetallionen aktiviert werden, (iii) hormoninduzierte Promotoren oder (iv) die Fusion des Gens mit der Hormonbindungsdomäne von Hormonrezeptoren, wie z.B. dem humanen Östrogenrezeptor.

Ein generelles Problem bei der Verwendung von Temperatur, Schwermetallionen und Hormonen zur Induktion der Genexpression besteht in den pleiotropen Effekten dieser Bedingungen bzw. Substanzen. Eine erste Forderung an ein konditionales Genexpressionssystem besteht daher darin, daß die induzierenden Bedingungen möglichst keine unerwünschten Nebenwirkungen besitzen.

Weitere Systeme beruhen auf der Adaptation prokaryonter Systeme an eukaryonte Zellen, wie z.B. die Fusion des lac-Repressors mit dem Herpes-Virus-Gen "virion protein 16" (VP16).

Von Gossen und Bujard (1992) wurde ein System beschrieben, das auf der Tetrazyklin (Tc)-abhängigen Bindung des Tet-Repressors (tetR) an seine Operatorsequenz (tetO) basiert. Durch Fusion des tetR mit der Transaktivierungsdomäne des Hepes simplex-Gens VP16 wurde ein Transaktivator (tTA) erzeugt, der in Abwesenheit von Tc an tetO bindet und in der Umgebung befindliche Promotoren aktiviert. In Anwesenheit von Tc wird die Bindung von tTA an tetO aufgehoben und damit die Promotoraktivierung unterbrochen. tetO wird bevorzugt in 7-facher Kopienanzahl angewandt und dann als "tetO7" bezeichnet. Dieses System erfordert für die konditionale Expression eines Genes von Interesse (GOI) eine intakte Expressionskassette für tTA sowie eine intakte Transkriptionseinheit für das GOI, bestehend aus einem Promotor, der tetO enthält, dem Leserahmen des Gens und einer Polyadenylierungsstelle (PA).

Dieses System funktioniert nur dann, wenn tTA in ausreichender Menge während der Untersuchungszeit exprimiert wird und wenn die Expressionskassette für das GOI intakt, zugänglich und damit aktivierbar durch tTA ist.

Dieses Verfahren, das auch in der WO 94/29442 beschrieben ist, benutzte integrierende Expressionsvektoren, um diese Ziele zu erreichen. Integrierende Expressionsvektoren besitzen jedoch eine Reihe von Nachteilen:
- In manchen Zielzellen kommt es nach physikalischem DNA-Transfer nicht oder nur sehr selten zu einer Integration des Konstruktes in das Genom der Zielzelle;
- das Ausmaß der Expression wird in besonderem Maße durch den Integrationsort bestimmt ("Positionseffekt").

Diese beiden Nachteile wurden bisher dadurch überwunden, daß eine Vielzahl von Transfektanten mit unterschiedlichen Integrationsstellen etabliert und anschließend durch Testung der Tc-abhängigen Genexpression nach in der Regel transienter Transfektion eines tetO7-kontrollierten Reportergens die optimale Zelle ermittelt wurde. Diese Zelle diente dann als Zielzelle für einen weiteren stabilen Transfektionsansatz mit einem Expressionsplasmid für das GOI unter der Kontrolle eines tetO-enthaltenden Promotors. Die stabilen Transfektanten wurden dann einer Einzelzellklonierung unterworfen und die einzelnen Klone anschließend auf das Expressionsniveau des GOI in An- und Abwesenheit von Tc untersucht.

Um einen Zellklon zu ermitteln, der das GOI in erwünschter Weise konditional exprimiert, ist es daher unter Umständen erforderlich, 10 bis 100 Zellklone zu untersuchen. Dieses Verfahren garantiert jedoch nicht zwingend einen stabilen Klon, der sich auch nach mehreren Passagen in Zellkultur immer noch so verhält wie bei der ersten Untersuchung. Die Erfahrung zeigt, daß sich die Induzierbarkeit sowie das maximale Expressionsniveau nach unterschiedlich langer Zeit in Kultur wieder verschlechtert. Die Mechanismen, die diesen Veränderungen zugrunde liegen, sind noch nicht vollkommen verstanden. Mögliche Erklärungen sind: Methylierung der Promotorregion, Rekombination bzw. Verlust eines Teils der Plasmide, Annahme einer Chromatinkonfiguration, die für Transkriptionsfaktoren unzugänglich ist.

Anforderungen an ein optimales konditionales Genexpressionssystem:
- keine Nebenwirkungen der Induktions- oder Abschaltungsbedingungen;
- die Menge des zu regulierenden Genproduktes sollte uninduziert möglichst gering sein ("leakiness");
- Regulierbarkeit möglichst über mehrere Größenordnungen.

Es ist eine Aufgabe der vorliegenden Erfindung, einen Vektor bereitzustellen, der die aus dem Stand der Technik bekannten Nachteile vermeidet. Diese erfindungsgemäß bereitzustellenden Vektoren sollen u.a. auch ohne Selektion in den Zellen besser "zurückgehalten" werden, eine geregelte Expression frei von Positionseffekten erlauben und ein wesentlich vermindertes Risiko der Insertionsmutagenese besitzen.

Diese Aufgabe wird erfindungsgemäß durch den im Anspruch 1 näher gekennzeichneten Vektor gelöst. Bevorzugte Ausführungsformen der Erfindung ergeben sich aus den Unteransprüchen.

In einer weiteren Ausführungsform der Erfindung wird ein Verfahren zur konditionalen Expression eines oder mehrerer Gene von Interesse unter Verwendung des erfindungsgemäß bereitgestellten Vektors zur Verfügung gestellt.

Nachfolgend wird die Erfindung anhand bevorzugter Ausführungsformen und anhand von Ausführungsbeispielen sowie der beiliegenden Abbildungen näher beschrieben.

Die Erfindung wird nachfolgend insbesondere in Verbindung mit EBV-Nukleotidsequenzen beschrieben, die eine episomale Replikation gestatten; selbstverständlich sind auch andere derartige Sequenzen einsetzbar, die die erfindungsgemäß erforderliche episomale Funktionalität gestatten.

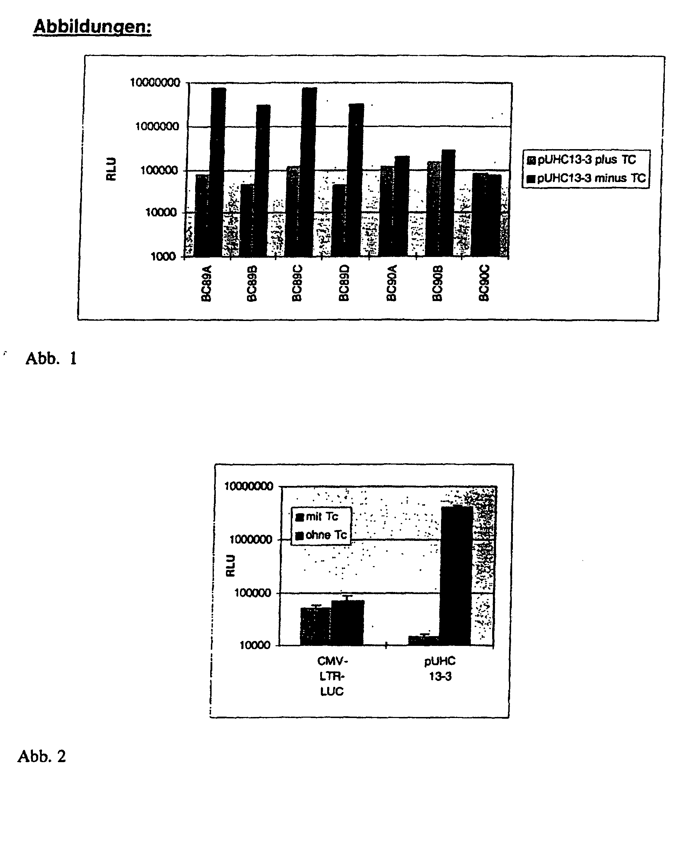

Die Abbildungen 1 bis 29 zeigen graphische Darstellungen der Versuchsergebnisse der Beispiele sowie Vektoren, die in den nachfolgenden Beispielen näher beschrieben werden.

Erfindungsgemäß wird somit ein in Eukaryonten episomal replizierender Vektor zur konditionalen Genexpression in Eukaryonten bereitgestellt, enthaltend, in funktioneller Verbindung, auf einem DNA-Molekül, zumindest eine Polynukleotidsequenz, die für ein mit Tetrazyklin oder seinen Derivaten oder Analogen regulierbares Transaktivator-Fusionsprotein (tTA) kodiert, wobei tTA einen prokaryontischen Tet-Repressor und einen transkriptions aktivator umfaßt, einen tTA-responsiblen Promotor, der die tet-Operator-Sequenz (tetO) enthält, an die tTA binden kann, eine Polynukleotidsequenz, die für mindestens ein Gen von Interesse (GOI) kodiert, eine Polyadenylierungsstelle (PAA), eine Polynukleotidsequenz für zumindest einen selektierbaren Marker, und Polynukleotidsequenzen, die eine episomale Replikation des Vektors in Eukaryonten bewirken.

In der Erfindung sind die obenbeschriebenen Elemente des Vektors auf einem einzigen Vektormolekül vereinigt.

Der Tet-Repressor von tTA kann bevorzugt ein von Tn10-stammender Tet-Repressor sein.

Das Polypeptid von tTA, das die Transkription in eukaryontischen Zellen direkt oder indirekt aktiviert, kann aus dem Herpes simplex-Virus-Virionprotein 16 stammen.

Es kann aus mindestens einem Element der Gruppe saurer, prolinreicher, serin/threonin-reicher und glutaminreicher Transkriptions-Aktivatorpolypeptide ausgewählt sein.

Das Polypeptid von tTA, das die Transkription in eukaryontischen Zellen direkt oder indirekt aktiviert, kann eine Interaktionsdomäne sein, ausgewählt aus mindestens einem Element der Gruppe, bestehend aus einer Leucin-Zipper-Domäne, einer Helix-Loop-Helix-Domäne und einer Zinkfinger-Domäne. In einer Ausführungsform der Erfindung handelt es sich um eine Interaktionsdomäne aus einem TATA-Bindungsprotein.

Die für tTA kodierende Polynukleotidsequenz weist einen Promotor auf, der bevorzugt aus einem Element der Gruppe ausgewählt ist, bestehend aus CMV-, tk- und myc-Promotoren.

Der durch Tetrazyklin regulierbare Transkriptionsaktivator ist somit ein Fusionsprotein (bezeichnet als tTA), das aus zwei Polypeptiden zusammengesetzt ist. Das erste Polypeptid ist ein tet-Repressor, der an tet-Operatorsequenzen binden kann. In einer Ausführungsform der Erfindung bindet er in Abwesenheit von Tetrazyklin, nicht jedoch in Anwesenheit von Tetrazyklin. In einer weiteren Ausführungsform der Erfindung bindet der Tet-Repressor an tet-Operatorsequenzen in Anwesenheit von Tetrazyklin, nicht dagegen in Abwesenheit von Tetrazyklin. Das zweite Polypeptid aktiviert direkt oder indirekt die Transkription in Eukaryontenzellen. Ein bevorzugtes Beispiel für das zweite Polypeptid ist die Transkriptionsaktivatordomäne des Herpes simplex-Virus-Virion-Proteins 16.

Zur Offenbarung des Tet-Repressor/Operator/Induktor-Systems wird auf die WO 94/29442, auf die Veröffentlichung von Gossen und Bujard in Proc. Natl. Acad. Sci., Band 89, S. 5547-5551 und auf die Veröffentlichung von Gossen et al. in Science, Band 268, S. 1766-1769 hingewiesen.

Die für tTA kodierende Polynukleotidsequenz steht bevorzugt unter Kontrolle eines konstitutiv aktiven Promotors, der bevorzugt ausgewählt wird aus einem Element der Gruppe, bestehend aus CMV-, tk- und c-myc-Promotoren, oder die tTA-kodierende Polynukleotidsequenz steht unter Kontrolle eines tTA-responsiblen Promotors (Autoregulation).

Der tTA-responsible Promotor besteht mindestens aus einer tet-Operatorsequenz und einem Promoter, bevorzugt einem Minimalpromotor, der die Transkription des Gens von Interesse initiiert. Unter einem "Minimalpromotor" ist eine Teilpromotorsequenz zu verstehen, die den Transkriptionsstart definiert, die aber selbst allein nicht ausreichend in der Lage ist, die Transkription wirksam zu initiieren. Die Aktivität derartiger Minimalpromotoren hängt von der Bindung von Aktivatoren an funktionell damit verbundene Bindungsstellen ab, beispielsweise von der Bindung des tetrazyklin-regulierten Transaktivators.

Der tTA responsible Promotor umfaßt zumindest eine Sequenz mit Promotorfunktion und eine Sequenz mit Operatorfunktion, an die tetR binden kann.

Der tTA-responsible Promotor enthält in einer bevorzugten Ausführungsform der Erfindung den TP-Promotor des Epstein-Barr-Virus (EBV).

In einer Ausführungsform der Erfindung umfaßt der tTA-responsible Promotor den TP-Promotor von EBV, bei dem das EBNA2-responsible Element durch die tet-Operator-Sequenz (tetO) ersetzt oder ergänzt wurde. Die Funktion des EBNA2-responsiblen Elements ist bekannt (vgl. beispielsweise Zimber-Strobl 1993, 1994).

Der tTA-responsible Promotor liegt in Verbindung mit der tet-Operator-Sequenz (tetO) vor. tetO liegt bevorzugt in multipler Kopienanzahl vor, insbesondere bevorzugt in einer 7-fachen Kopie.

Der tTA-responsible Promotor umfaßt somit die tet-Operator-Sequenz und einen Promotor zur Initiation der Transkription des Gens von Interesse, wobei es sich hier bevorzugt um einen Minimalpromotor handelt.

Das Gen von Interesse, das durch den erfindungsgemäßen Transaktivator steuerbar ist, kann für ein humanes, ein virales oder ein bakterielles Protein kodieren. Beispielsweise kann das Gen von Interesse für ein Polypeptid kodieren, ausgewählt aus mindestens einem Element der Gruppe, bestehend aus Transkriptionsfaktoren, Onkogenen, Signalübertragungsmolekülen, Enzymen, Cytokinen, Zelladhäsionsproteinen, Tumorantigenen, co-stimulatorischen Signalmolekülen und ein nicht mit dem Empfänger identisches HLA. Bei den Cytokinen kann es sich um Interleukine, Interferone, kolonie-stimulierende Faktoren, Lymphokine und Wachstumsfaktoren handeln.

Die durch den erfindungsgemäßen Vektor exprimierten Proteine sind häufig medizinisch wertvolle Proteine, die entweder aus den Zellen isoliert werden oder in den Zellen verbleiben, so daß sie in vivo therapeutisch wirksam sind.

Der erfindungsgemäße Vektor enthält einen selektierbaren Marker, die an sich aus dem Stand der Technik bekannt sind. Beispiele hierfür sind Polynukleotidsequenzen, die für ein Tk-Protein, ein Neomycin-Protein oder ein Hygromycin-Protein kodieren.

Der erfindungsgemäße Vektor enthält zwingend solche Polynukleotidsequenzen, die für die episomale Replikation des Vektors in Eukaryonten verantwortlich sind. Der erfindungsgemäße Vektor enthält aber auch solche Polynukleotidsequenzen, die eine Replikation des Vektors in Prokaryonten ermöglichen, so daß es sich bei dem erfindungsgemäßen Vektorssystem um einen Shuttle-Vektor handelt.

Die für eine episomale Replikation des erfindungsgemäßen Vektors in Eukaryonten verantwortlichen Polynukleotidsequenzen umfasen in einer Ausführungsform der Erfindung den Replikationsursprung (ori) von EBV (oriP) oder von BPV (Bovines Papilloma-Virus). Zur Aufrechterhaltung der episomalen Replikation bei Verwendung des EBV-Systems ist neben dem oriP weiterhin eine Polynukleotidsequenz notwendig, die für EBNA1 kodiert.

### Verpackung: Verpackbarkeit der Vektoren in EBV Partikel

Das Genom des EBV liegt in infizierten Zellen grundsätzlich in zwei Zuständen vor: dem Zustand der Latenz und der Virusproduktion (lytischer Zustand). Der letztere Zustand ist gekennzeichnet durch die Vervielfältigung der viralen DNA (Replikation) und die Expression einer Vielzahl von viralen Genprodukten, wie z.B. der Strukturproteine der viralen Partikel. Die lytische Replikation der viralen DNA beginnt an definierten DNA-Strukturen: Den sogenannten "lytic origins of DNA replication", die dupliziert im Genom des Virus vorliegen (orilyt) (Hammerschmidt et al., 1988). Die Amplifikation der viralen DNA durch Replikation ist Voraussetzung für eine effiziente Verpackung. Weiterhin werden für die Verpackung in virale Partikel noch die Enden der viralen DNA, die sogenannten "terminalen Repetitionen" (TR) benötigt (Hammerschmidt et al., 1989). Die Erweiterung der erfindungsgemäßen Vektoren um diese beiden Strukturen (orilyt und TR) erlaubt die Verpackung in virale Partikel. Nach Transfer der Vektoren in eine EBV positive Zelle und der Induktion des lytischen Zyklus werden diese in Form von Multimeren in infektinöse Partikel verpackt. Diese Partikel können dann zur Infektion von den EBV-Rezeptor tragenden Zellen zum Einsatz kommen. Dieses Vorgehen kann dann zum Einsatz kommen, wenn sich die gewünschten Zielzellen nicht mit den herkömmlichen biophysikalischen Methoden transfizieren lassen.

Der erfindungsgemäße Vektor kann verwendet werden, um therapeutisch wertvolle Gene, beispielsweise Cytokin-Gene oder Tumorantigene, in Zellen, bevorzugt B-Zellen, einzuschleusen. Weiterhin sind die erfindungsgemäßen Vektoren zur Therapie maligner und viraler Erkrankungen des Menschen, insbesondere von Erkrankungen, die mit Tumoren der B-Zellen einhergehen, einzusetzen. Die erfindungsgemäßen Vektoren eigenen sich insbesondere zur gentherapeutischen Anwendung.

Die erfindungsgemäß definierten Vektoren können auch als Genkonstrukte bezeichnet werden.

Die Erfindung verwendet in einer bevorzugten Ausführungsform das System der tetrazyklin-abhängigen Genregulation zusammen mit EBV-Vektoren, die eine episomale Replikation gestatten.

Das Genom des EBV liegt in der infizierten Zelle in der Regel episomal mit einer Kopienzahl zwischen 10 und 100 vor. Im Zustand der Latenz geht die Replikation des viralen Genoms von einem Replikationsursprung (oriP) aus (Ytes et al., 1984). Zur Aufrechterhaltung der episomalen Replikation ist weiterhin die Bindung des EBNA1-Proteins an den oriP (Yates et al., 1985) notwendig. Die von EBV abgeleiteten Vektoren bestehen aus beispielsweise pBR-Sequenzen oder anderen Plasmidsequenzen, oriP, einer Expressionskassette für EBNA1 und einem Selektionsmarker für eukaryonte Zellen (z.B. das Hygromyzinresistenzgen). Diese Vektoren besitzen darüber hinaus noch die Kapazität für 20 bis 30 kb weitere Fremd-Sequenzen. Konstrukte, die auf diesen Vektoren basieren, werden in der Zelle: (i) auch ohne Selektion sehr viel besser "zurückgehalten" (Middleton and Sugden, 1994), (ii) erlauben eine geregelte Expression frei von Positionseffekten und (iii) besitzen ein wesentlich vermindertes Risiko der Insertionsmutagenese. Die Effizienz, mit diesen Vektoren stabil transfizierte Zellen zu erhalten, ist im Vergleich zu reinen Plasmidvektoren wesentlich höher. Wir konnten zeigen, daß mit Hilfe dieser Vektoren transfizierte regulierbare Genkonstrukte über einen langen Zeitraum ihre Induzierbarkeit behielten (Polack et al., 1992; Bouvier et al., 1993).

In einer bevorzugten Ausführungsform der Erfindung wird des erfindungsgemäße Vektor in EBV-positive Zellen eingebracht. EBV-positive Zellen exprimieren diejenigen Proteine, die für eine episomale Replikation des erfindungsgemäßen Vektorsystems notwendig sind. Es ist in diesem Fall nicht notwendig, beispielsweise daß EBNA1-Protein durch den erfindungsgemäße Vektor exprimieren zu lassen.

Die erfindungsgemäß verwendeten, in der Zielzelle episomal replizierenden Vektoren vermeiden die aus dem Stand der Technik bekannten Schwierigkeiten, die mit einer stabilen Expression mit integrierenden Vektoren verbunden sind. Um dies für die Expression der tetrazyklin-abhängigen Transaktivatoren zu zeigen, wurde zunächst die Expressionskassette für tTA (konstitutiver Promotor, Polynukleotidsequenz, die für ein Gen kodiert (hier: tTA) und Polyadenylierungsstelle) unter der Kontrolle des Cytomegalie-Virus "immediate early gene"-Promotors (CMV) bzw. des Thymidinkinase (tk)-Promotors in vom Epstein-Barr-Virus (EBV) abgeleiteten Vektor pHEBOPL einkloniert. pHEBOPL ist ein Derivat (Polack et al., 1991) des von Sugden et al. (1985) beschriebenen Vektors.

### Konstruktionsbeschreibung:

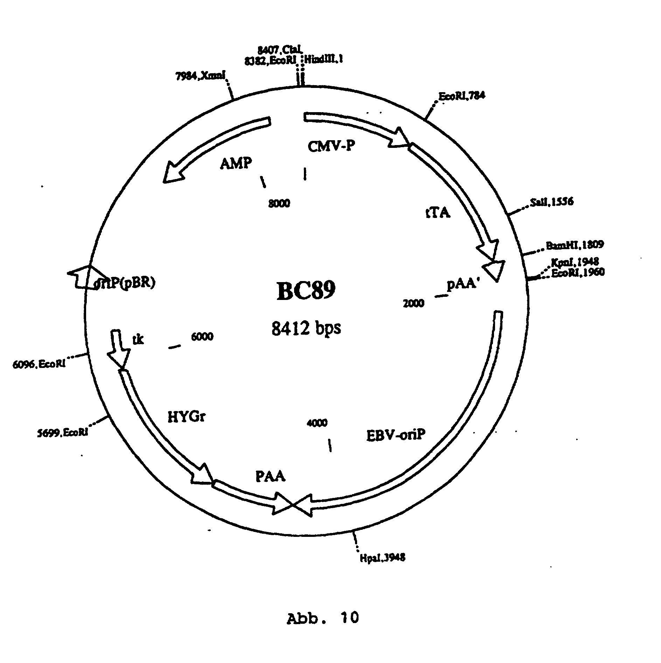

### BC89-Konstruktionsbeschreibung (Abb. 10)

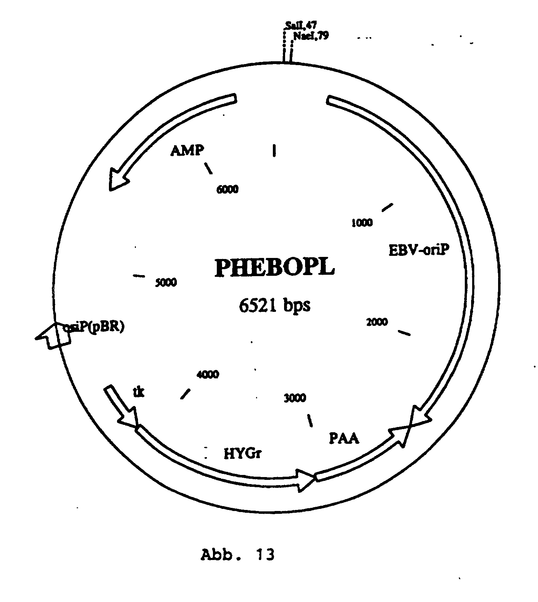

Der CMV-Expressionsvektor für tTA pUHD15-1 (Gossen und Bujard, 1992) wurde als XhoI und HpaI-Fragment (Sequenzposition 1 bis 1924) in die Restriktionsenzymspaltstellen SalI und NaeI (Sequenzposition 47 bis 79) des Vektors pHEBOPL (Abb. 13) (Erstbeschreibung: (Polack et al., 1991) einkloniert.

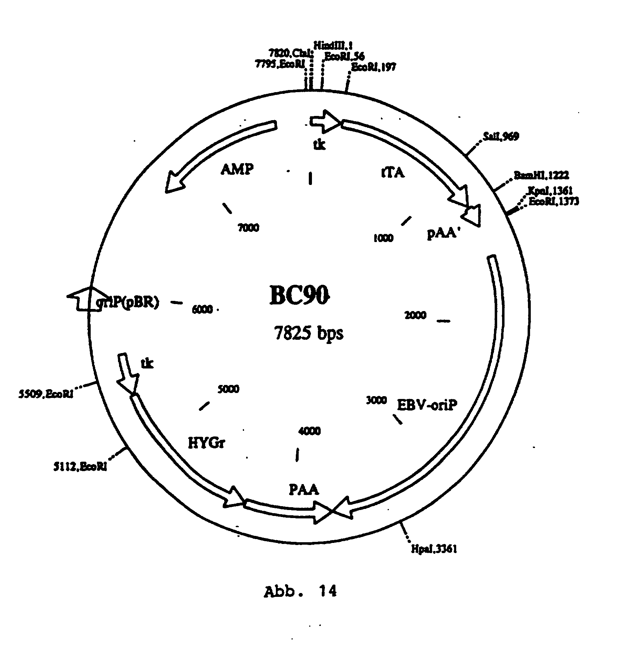

### BC90-Konstruktionsbeschreibung (Abb. 14)

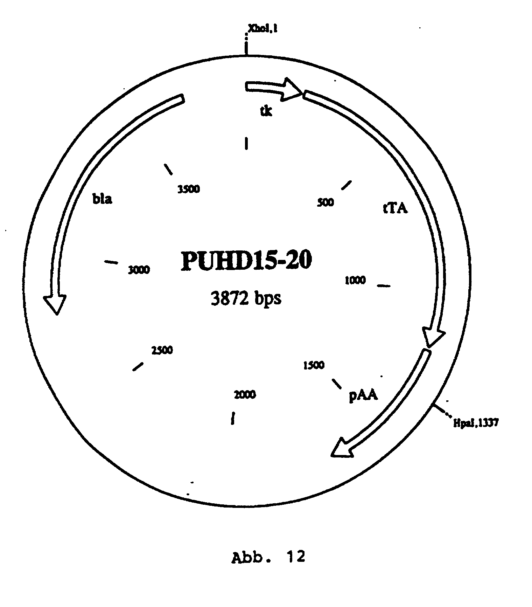

Der tk-Expressionsvektor für tTA pUHD15-20 (Abb. 12) (Gossen und Bujard, 1992) wurde als XhoI und HpaI-Fragment (Sequenzposition 1 bis 1924) in die Restriktionsenzymspaltstellen SalI und NaeI (Sequenzposition 47 bis 79) des Vektors pHEBOPL einkloniert (BC90).

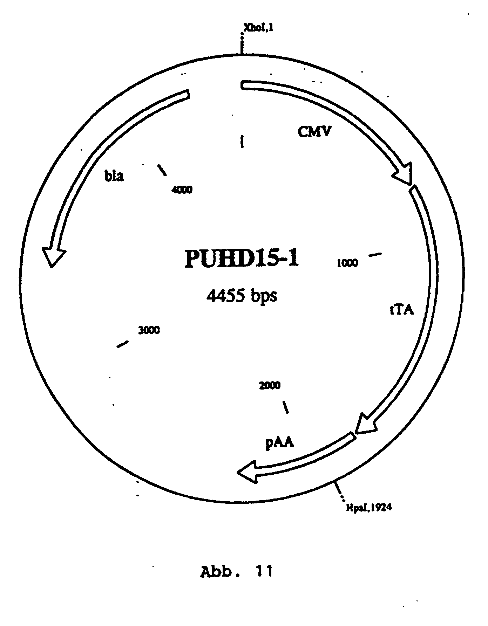

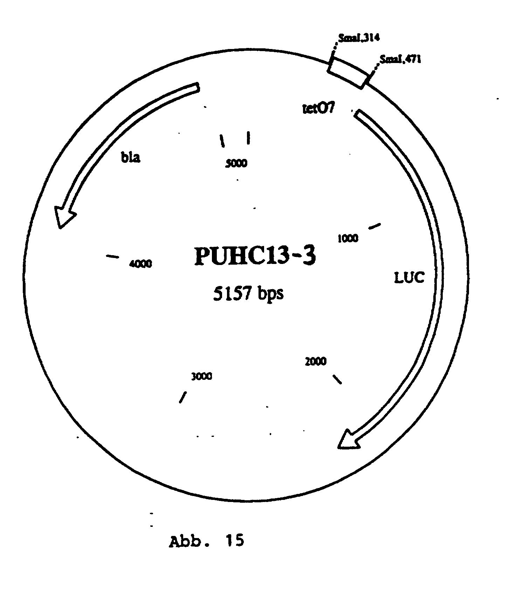

Die Expressionsplasmide BC89 (Abb. 10) und BC90 (Abb. 14) wurden zunächst transient in Raji-Zellen zusammen mit pUHC13-3 (Abb. 15) vgl. Gossen und Bujard, 1992) transfiziert. Die Transfektionsansätze wurden anschließend für 48 Stunden mit und ohne Tetrazyklin (TC) 1 µg/ml behandelt. Die Messung der Luziferaseaktivität in Extrakten von diesen Zellen zeigte, daß die Plasmide BC89 und BC90 ebenso tTA exprimierten wie die Ausgangsplasmide pUHD15-1 (Abb. 11) und pUHD15-20 (Abb. 12) (Daten nicht gezeigt).

Weiterhin wurde die Burkitt-Lymphom-Zellinie Raji mit den Plasmiden BC89 und BC90 transfiziert. Durch Selektion mit Hygromycin wurden mehrere Sublinien (A - D) etabliert.

Durch transiente Transfektion von pUHC13-3 (Abb. 15) (CMV-tetO7-LUC) in die so entstandenen Sublinien wurde die stabile Expression des Tc-abhängigen Transaktivators nachgewiesen (Abb. 1).

Dieses Experiment zeigt, daß in Lymphomzellen durch stabile Transfektion eines episomal replizierenden Expressionsvektors funktionelle Mengen von tTa exprimiert werden können. Die vier bzw. drei verschiedenen Transfektanten mit BC89 bzw. BC90 zeigen untereinander keine Unterschiede hinsichtlich der Basisaktivität des CMV-tetO7-Promotors oder dessen Induzierbarkeit. Weiterhin zeigt das Experiment, daß der tk-Promotor (BC90) keine ausreichende Expression des Transaktivators erlaubt. Durch Tc wird in diesen Zellen (BC90A-C) die Luziferase-Expression lediglich um den Faktor 2 moduliert im Gegensatz zum 60- bis 100-fachen Unterschied in den CMV-tTA tragenden Zellinien (BC89A-D). Das Experiment belegt weiterhin, daß die Basisaktivität (d.h. minus TC) des pUHC13-3-Konstruktes durch die Menge an tTA nicht wesentlich beeinflußt wird.

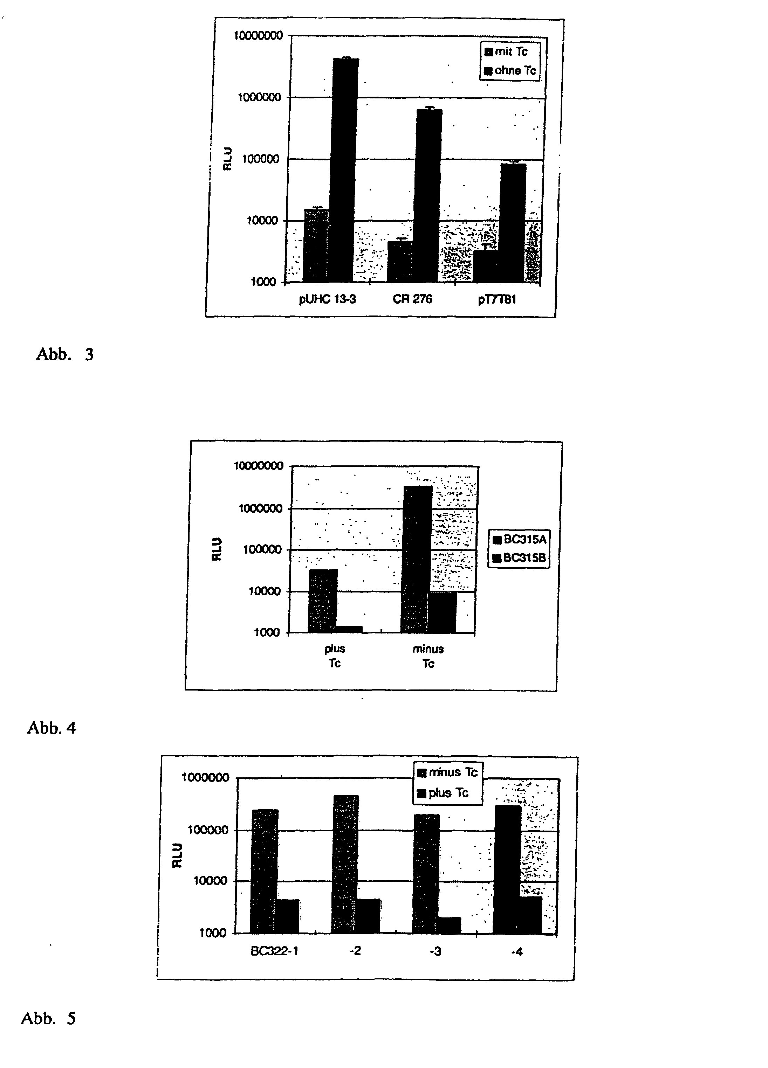

Der Promotor von CMV-tetO7-LUC (pUHC13-3) war im uninduzierten Zustand (ohne Tc) etwa ein Drittel so aktiv wie der Standardexpressionsvektor CMV-LTR-LUC (Abb. 2). Im induzierten Zustand war pUHC13-3 sogar ca. 90-fach stärker als CMV-LTR-LUC. Für funktionelle Analysen erschien die "Basalaktivität" sowie die maximale Expression wesentlich zu hoch. Der ebenfalls von Gossen und Bujard hergestellte tk-Promotor mit tetO7 (pT7t81) zeigte im Vergleich zu pUHC13-3 zwar eine geringere Basalaktivität, aber die Aktivierbarkeit war mit Faktor 26 am schlechtesten im Vergleich mit den anderen getesteten Promotoren (Abb. 3).

Ein bevorzugtes Ziel der vorliegenden Erfindung ist der Einsatz der erfindungsgemäß episomal replizierenden Vektoren in B-Zellen.

Die beiden bisher getesteten Promotoren erschienen für den Gebrauch in B-Zellen ungeeignet. Es wurde daher der TP-Promotor des EBV als Basis für die Konstruktion eines Tc-regulierbaren Expressionsvektors gewählt. Dieser Promotor wird in EBV-positiven B-Zellen äußerst effizient durch den von EBV kodierten Transkriptionsfaktor EBNA2 kontrolliert (Zimber-Strobl et al., 1991). Daß das DNA-Element im TP-Promotor an das EBNA2 im Komplex mit RBPJκ bindet, ist bekannt (Zimber-Strobl et al., 1993; Meitinger et al., 1994). Die Regulation der Promotoraktivität wird durch den DNA-bindenden, zellulären Transkriptionsfaktor RBPJκ vermittelt (Zimber, Strobl et al., 1994). Da der TP-Promotor ohne EBNA2 vollkommen inaktiv ist und durch EBNA2 um mehrere Größenordnungen aktiviert werden kann, wurde das ENBA2-responsible Element in diesem Promotor durch das tetO7-Element aus pUHC13-3 ersetzt.

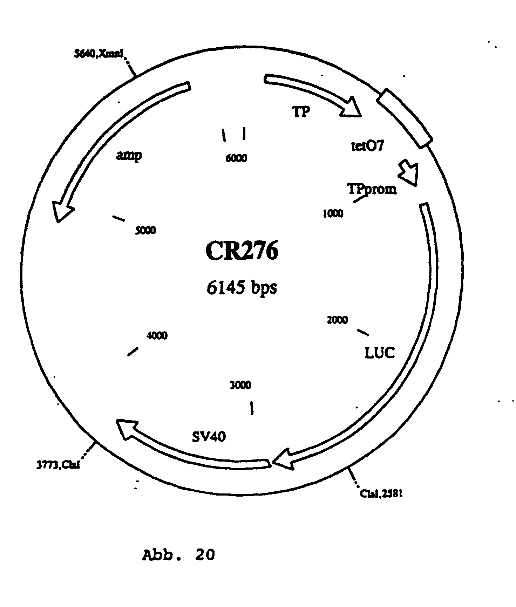

### CR276-Konstruktionsbeschreibung (Abb. 20)

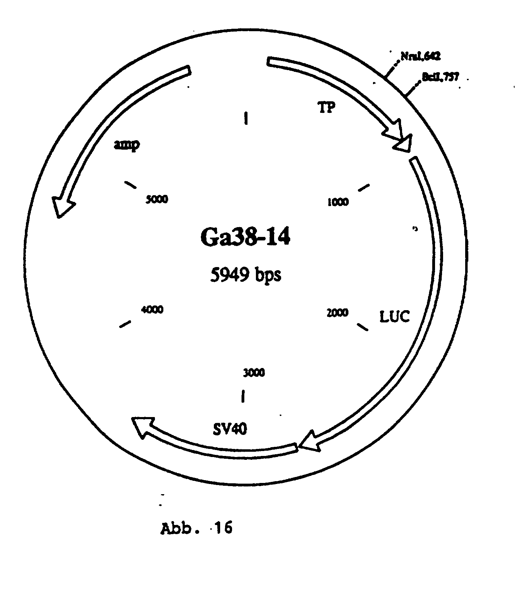

Die EBNA2-responsible Stelle im TP-LUC-Promotorkonstrukt Ga38-14 (Abb. 16) wurde über die Restriktionsenzymschnittstellen NruI und BclI entfernt. Die überstehenden Enden wurden durch Behandlung mit Klenow-Polymerase aufgefüllt und anschließend das tet-Operon (tetO7) als SmaI-SmaI-Fragment aus dem Ursprungsklon (pUHC13-3) (Abb. 15) von Gossen und Bujard (1992) eingesetzt.

Das TPtetO7-Luc-Konstrukt CR276 (Abb. 20) wurde transient in die tTA-exprimierende Raji-Zellinie BC89C transfiziert (Abb. 3). Der Vergleich mit pUHC13-3 zeigt, daß die "Basalaktivität" um den Faktor 4 bis 5 und die maximale (induzierte) Aktivität um den Faktor 6 bis 7 niedriger war.

### Die erfindungsgemäße "all in one"-Strategie

Das von Gossen und Bujard (1992) vorgeschlagene System zur konditionalen Genexpression sieht vor, daß zunächst eine den Transaktivator (tTA) produzierende Zellinie hergestellt wird. Die optimale Zellinie wird durch transiente Transfektion geeigneter Reportergenkonstrukte ermittelt. In einem zweiten Transfektionsansatz soll dann das GOI unter der Kontrolle des tet-Operons in diese Zelle eingeführt werden. Durch Testung verschiedener Subklone soll die geeignete, das GOI konditional exprimierende Zellinie identifiziert werden. Dieses Vorgehen setzt voraus, daß es möglich ist, mit Hilfe integrierender Vektoren eine größere Anzahl stabiler Transfektanten einer bestimmten Zellinie herzustellen, die über längere Zeit konstante Mengen des tTA-Moleküls produzieren. Weiterhin muß das GOI-Konstrukt auf solche Weise in das Wirtsgenom integrieren, daß es über längere Zeit für tTA zugänglich ist.

Dieses Vorgehen scheitert jedoch nicht selten an der Transfizierbarkeit von bestimmten Zellen (z.B. B-Zellen bzw. von B-Zellen abgeleitete Tumorzellen). Ein weiteres Problem stellt die permanente stabile Expression von Fremdgenen dar. Es ist bekannt, daß integrierende Expressionsplasmide, selbst wenn sie über Retroviren in die Zelle eingebracht werden, in vielen Zelltypen nach einiger Zeit abgeschaltet werden (Xu et al., 1989).

Um das Problem der zweistufigen Transfektion zu umgehen, wurde daher erfindungsgemäß ein Plasmid erzeugt, das episomal repliziert, das tTA exprimiert und das ein GOI (hier beispielhaft das Luziferasegen) unter der Kontrolle des TP-tetO7-Promotors trägt. Mit Hilfe dieses Plasmids sollte es möglich sein, in einem Schritt eine konditionale Expression des GOI (hier der Luziferase) zu erreichen.

### BC315-Konstruktionsbeschreibung

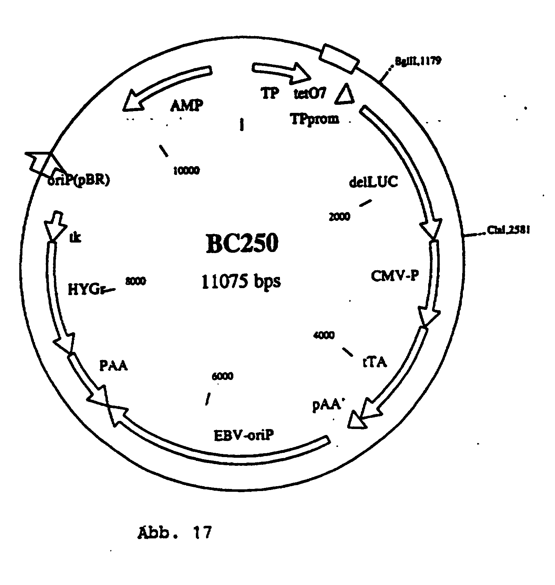

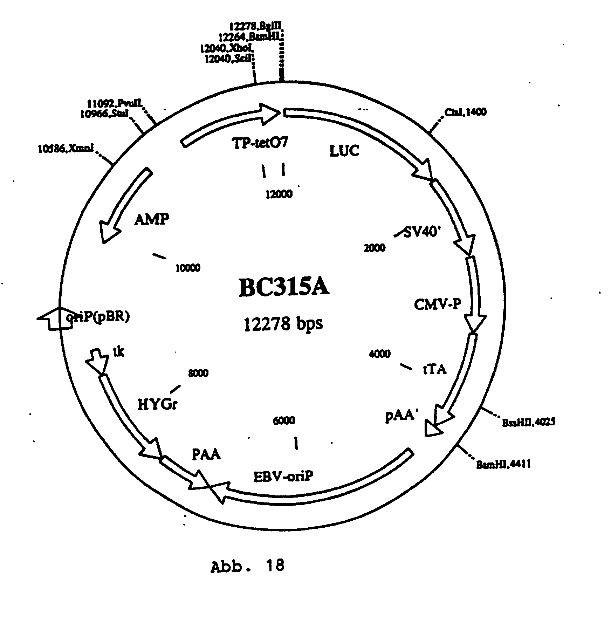

Zunächst wurden der TP-tetO7-Promotor und Teile des Luziferasegens als XmnI-ClaI-Fragment in BC89 ebenfalls über XmnI und ClaI einkloniert (BC250) (Abb. 17). Aus BC250 wurde das Luziferasegen über BglII und ClaI entfernt und ein BglII-Linker ("New England Biolabs" #1066) eingefügt (BC252). Das Luziferasegen wurde zusammen mit der SV40-Polyadenylierungsstelle als BamHI-BglII-Fragment aus BC311 in die BglII-Schnittstelle von BC252 einkloniert (BC315A) (Abb. 18). Als Kontrollplasmid wurde zusätzlich der Klon BC315B erzeugt, der das Luziferasegen in umgekehrter Orientierung enthält.

Es zeigte sich eine 100-fache Aktivierbarkeit des TPtetO7-Promotors auf dem Konstrukt BC315A. Dieses Ergebnis zeigte, daß weder die Vektoranteile (oriP, Hygromyzinresistenzgen) noch die tTA-Expressionskassette die Induzierbarkeit dieses Promotors beeinträchtigen. Es wurden daher stabile Raji-Transfektanten mit dem Plasmid BC315A etabliert und die Luziferaseaktivität vor und nach Tc-Behandlung gemessen (Abb. 5).

Das in Abb. 5 gezeigte Experiment zeigt die folgenden wesentlichen Merkmale des erfindungsgemäßen Vektors:
- Auch nach stabiler Transfektion und der damit verbundenen Ausbildung einer geordneten Chromatinstruktur des eingeführten Plasmids bleibt die Induzierbarkeit des Promotors erhalten. Die räumliche Nähe des CMV-Promotor/Enhancer-Bereichs, der die Expression des tTA-Moleküls gewährleistet, zum TPtetO7-Promotor führt nicht zu dessen unmodulierbarer konstitutiver Expression.
- Die vier unabhängig voneinander entstandenen Sublinien zeigen nur eine geringgradige Variation hinsichtlich der Basalaktivität und der Induzierbarkeit der Luziferaseaktivität.

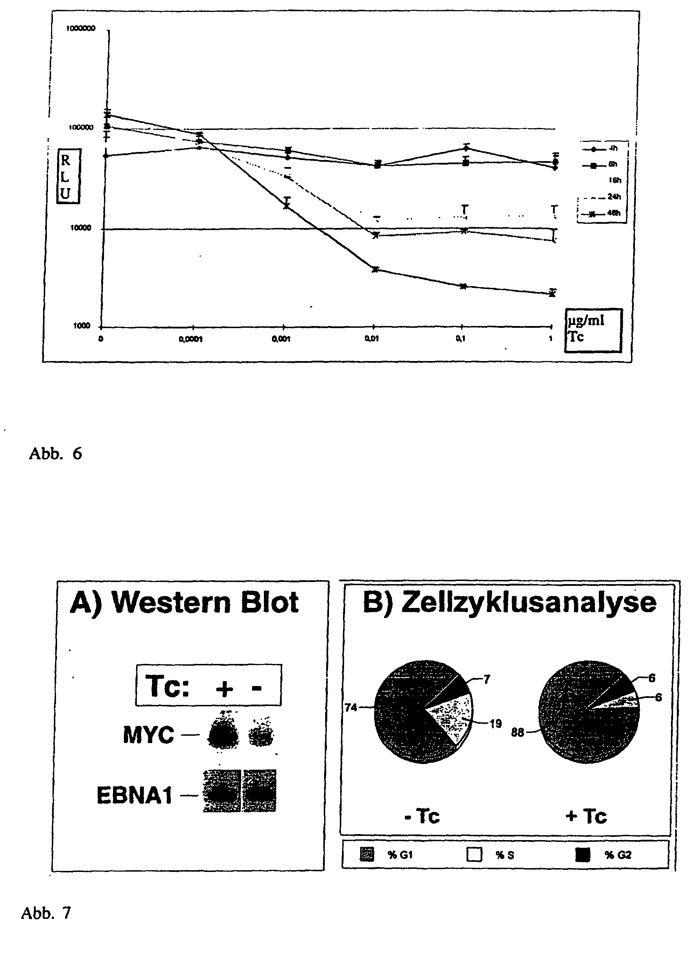

Die stabile Linie BC322-2 wurde hinsichtlich des zeitlichen Verlaufes und der Dosis-Wirkungsbeziehung genauer untersucht. Die Zellen wurden mit verschiedenen Konzentrationen an Tc behandelt und die Luziferaseaktivität nach 4, 8, 16, 24 und 48 Stunden bestimmt. Das Ergebnis des Experiments zeigt (Abb. 6), daß die maximale Wirkung nach 48 Stunden bei 1 µg/ml Tc erreicht wird. Da sowohl die Stabilität der Luziferase-RNA als auch des Proteins in dieses Experiment eingehen, kann über die tatsächliche Geschwindigkeit der Regulation der Promotoraktivität aufgrund dieses Experimentes keine Aussage getroffen werden. Eigene unveröffentlichte Beobachtungen der Stabilität des Luziferaseproteins haben gezeigt, daß die Halbwertszeit des Proteins mehrere Stunden beträgt. Es ist daher anzunehmen, daß die maximale Regulation der Promotoraktivität bereits innerhalb weniger Stunden nach Zugabe von Tc stattfindet.

### Konditionale Expression des c-myc-Gens mit Hilfe des vorgestellten Systems

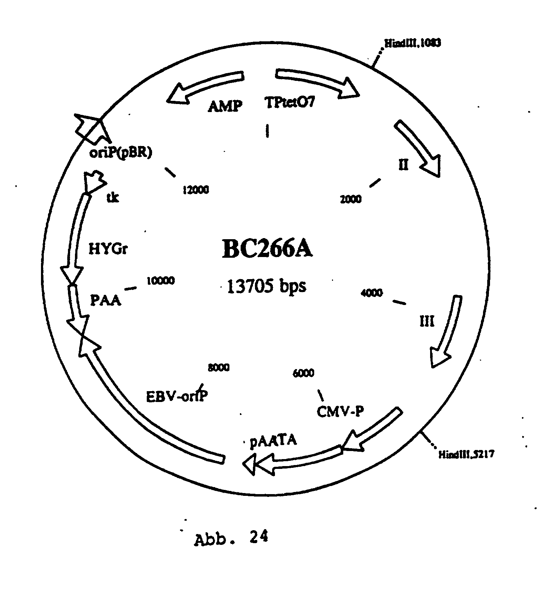

### BC266-Konstruktionsbeschreibung (Abb. 24)

In die BglII-Schnittstelle des Vektors BC252 (Abb. 22) wurde ein Teil des 1. Introns, das 2. und 3. Exon, sowie die Polyadenylierungsstelle des humanen c-myc-Gens (Sequenzposition 4077 bis 8082 nach (Gazin et al., 1984) als BamHI-BamHI-Fragment einkloniert. Das c-myc-Gen stammt aus dem Burkitt-Lymphom BL60 (Polack et al., 1991). Die BamHI-Schnittstelle bei NheI (Position 4077) wurde mit Hilfe eines Linkers eingeführt (BC253) (Abb 23). Die 3' von EcoRI (Sequenzposition von c-myc8082) gelegene BamHI-Schnittstelle stammt aus dem Polylinker des Vektors. Die konditionale Expression von Myc-Protein durch BC266 (Abb.24) wurde durch transiente Transfektion und anschließende Western Blot-Analyse geprüft (Daten nicht gezeigt).

Um die konditionale Expression von Myc durch BC266 näher zu untersuchen, wurde die Zellinie ER/EB2-5 (Kempkes et al., 1995) stabil mit BC266 transfiziert. Die Zielzelle ER/EB2-5 wurde gewählt, da die c-myc-Expression in dieser Zellinie unter der Kontrolle einer konditionalen Mutante des EBNA2-Gens steht. Die konditionale EBNA2-Mutante (ER-EBNA2) wurde durch Fusion mit dem Östrogen bindenden Teil des humanen Östrogenrezeptors erzeugt. Die Funktion von ER-EBNA2 ist abhängig von der Anwesenheit von Östrogen im Zellkulturmedium. In dieser Zelle läßt sich die endogene Myc-Expression durch Östrogenentzug fast vollständig ausschalten (Kempkes et al., 1995).

Eine Sublinie dieser Transfektion (BC493-6) wurde näher untersucht. Die Myc-Expression in dieser Zellinie kann durch die Zugabe von Tc zum Zellkulturmedium stark moduliert werden (Abb. 7). Die Tc vermittelte Abschaltung von Myc wird von einem Proliferationsstop begleitet. Das in Abb. 7 gezeigte Experiment wurde in Abwesenheit von Östrogen, d.h. bei abgeschalteter EBNA2-Funktion, durchgeführt. Das endogene c-myc-Gen ist in diesem Zustand fast nicht aktiv. Dieses Anwendungsbeispiel zeigt, daß mit Hilfe des Vektors BC252 eine konditionale Expression eines Nicht-Reportergens (c-myc) ereicht werden kann.

### Entwicklung von Vektoren, die durch Tetrazykline transkriptionell aktiviert werden

Gossen et al. (1995) haben eine Verbesserung ihres Systems vorgestellt. Der Tc-kontrollierte Transaktivator tTA wurde durch Mutagenese so verändert, daß er durch Tc nicht mehr reprimiert, sondern aktiviert wird. Dieses Molekül (rtTA) bindet nach Zugabe von Tc zum Zellkulturmedium an den Operator (tetO7).

### BC252rev Konstruktionsbeschreibung

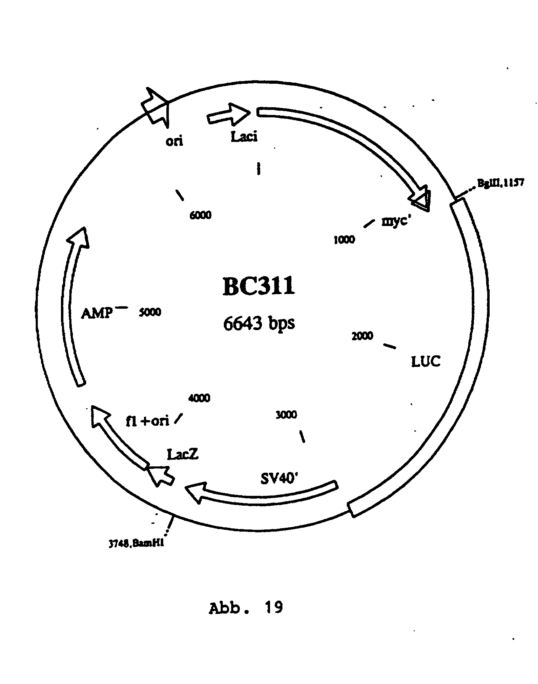

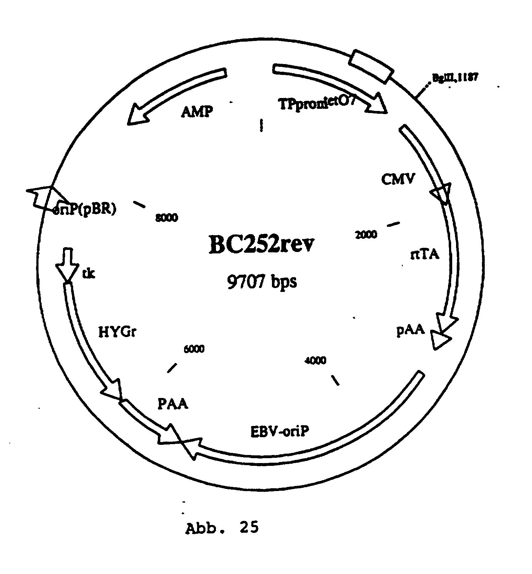

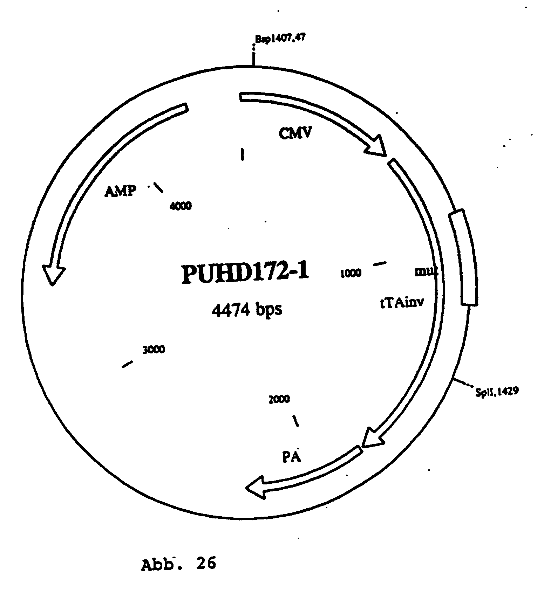

In BC252 wurden über BspI407 und SplI große Teile des CMV-Promotors und des kodierenden Bereichs für tTA durch rtTA aus pUHD172-1 (Abb. 26) ebenfalls über BspI407 und SplI ausgetauscht. In das so entstandene Plasmid BC252rev (Abb. 25) wurde über BglII das Luziferasegen inklusive Polyadenylierungsstelle aus BC311 (Abb. 19) als BamHI-Fragment einkloniert (MS2a).

Mit MS2A wurden Raji-Zellen stabil transfiziert. Das Ergebnis der Testung von acht stabilen Transfektanten ist in Abb. 7 gezeigt.

Die beiden hier vorgestellten Vektoren (BC252 und BC252rev) sind geeignet, in EBV-positiven Zellen eine konditionale Expression von beliebigen Genen zu erreichen. Für EBV-negative Zellen muß EBNA-1 entweder über ein zweites Plasmid in der Zelle exprimiert werden oder die EBNA1-Expressionskassette muß noch in die Vektoren BC252 und BC252rev eingefügt werden.

### Konditionale Expression der Raf-Mutante BXB Konstruktionsbeschreibung BC392

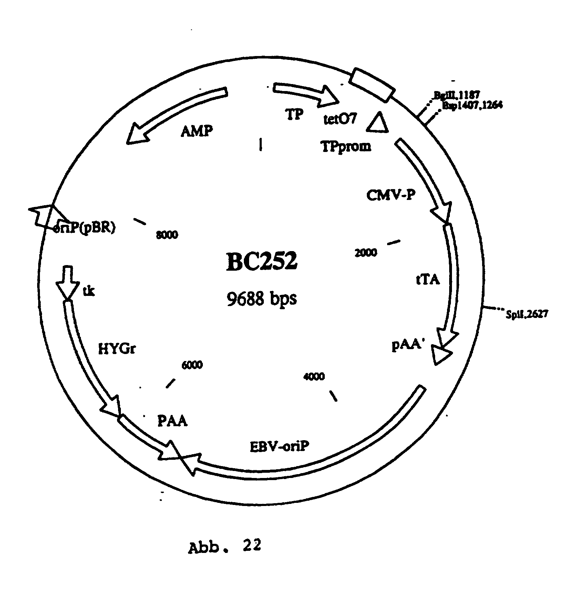

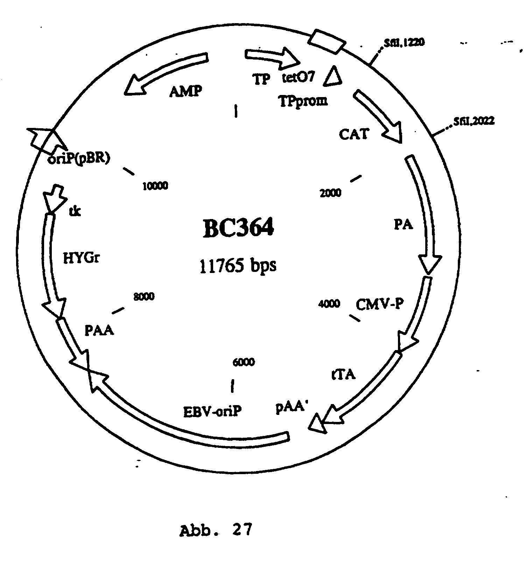

Die cDNA der Raf-Mutante BXB (Bruder et al., 1992) wurde als SfiI-SfiI-Fragment in die SfiI-Schnittstellen von BC364 einkloniert. BC364 (Abb. 27) ist ein Derivat von BC252 (Abb. 22), das zusätzlich das CAT-Gen (Chloramphenicolacetyltransferase) und eine Polyadenylierungsstelle enthält.

### Konditionale Expression von BXB in ER/EB2-5 Zellen

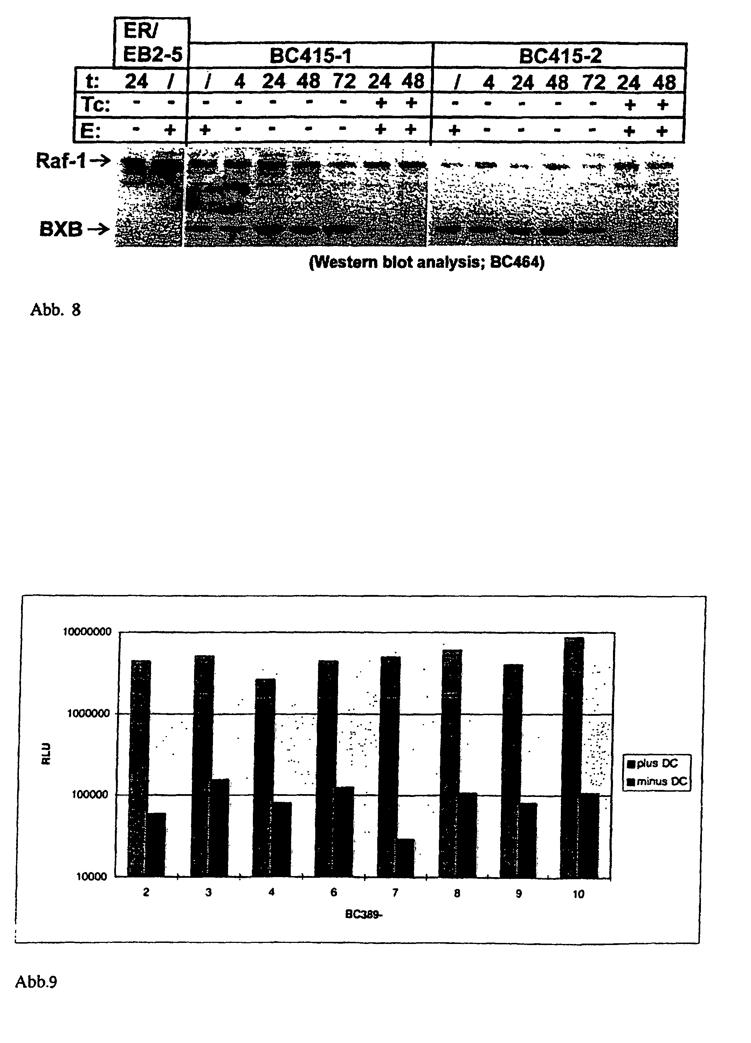

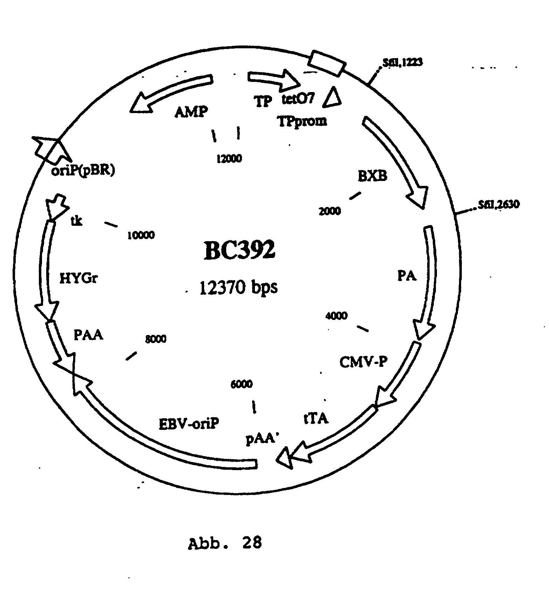

Das Konstrukt BC392 (Abb. 28) wurde in die Zellinie ER/EB2-5 transfiziert. Nach Selektion mit Hygromyzin wurden zwei stabile Linien etabliert; BC415-1 und BC415-2. Diese Zellinien wurden in An- und Abwesenheit von Östrogen und Tc für die in Abb. 8 angegebenen Zeiten kultiviert und Proteinextrakte gewonnen. Die Proteinextrakte wurden mit Hilfe der Western-Blot-Technik untersucht. Wie Abb. 8 zeigt, wird BXB in Abhängigkeit von Tc im Zellkulturmedium in BC 415-1 und -2 exprimiert wird.

Die ER/EB2-5-Zellen wachsen nur bei Anwesenheit von Östrogen im Zellkulturmedium (Kempkes et al., 1995). Östrogenentzug führt zu einem Proliferationsarrest. Die Expression des TPtet07-Promotors wird nicht vom Proliferationszustand der Zellen beeinflußt. Der Vektor ermöglicht daher auch eine Expression in ruhenden Zellen.

Die beiliegenden Abbildungen zeigen:
- Abb. 1:: Durch Tc modulierte Luziferase Aktivität in verschiedenen stabil mit BC89 bzw. BC90 transfizierten Raji Zellinien (BC89A-D und BC90A-C) nach transienter Transfektion von pUHC13-3 (CMV-tet07-LUC).
- Abb. 2:: Vergleich der Promotoraktivität von pUHC13-3 mit CMV-LTR-LUC nach transienter Transfektion in BC89C.
- Abb. 3:: Vergleich der Promotoraktivität der angegebenen Konstrukte nach transienter Transfektion in BC89C.
- Abb. 4:: Transiente Transfektion von BC315A und B in Raji.
- Abb. 5:: Luziferaseaktivität in verschiedenen Sublinien (BC322-1 bis -4) von stabil mit BC315A transfizierten Raji Zellen vor und nach Tc Behandlung.
- Abb. 6:: Die Sublinie BC322-2 (Raji x BC315A) wurde für die angegebenen Zeiten mit verschiedenen Konzentrationen Tc behandelt. Jeweils 3 verschiedene Ansätze wurden getrennt gemessen. Die verschiedenen Symbolpunkte geben den Mittelwert und die schwarzen senkrechten Linien die halbe Standardabweichung der Messungen an.
- Abb. 7:: Konditionale c-myc Expression in stabil mit BC266 transfizierten ER/EB2-5 Zellen (P493-6). A) Western Blot Analyse: Nachweis des Myc und EBNA1 Proteins in Extrakten von P493-6 Zellen, die für 48 Stunden mit (+) und ohne (-) Tc behandelt wurden. B) Zellzyklusverteilung: nach 48 Stunden Behandlung mit Tc von P493-6 Zellen befindet sich die Mehrzahl der Zellen in der G1 Phase.
- Abb. 8:: Konditionale BXB Expression in stabil mit BC392 transfizierten ER/EB2-5 Zellen (BC415-1 und -2). Western Blot Analyse: Nachweis des BXB Proteins in Extrakten von BC415-1 und -2 Zellen, die für 24 und 48 Stunden mit (+) und ohne (-) Tc bzw. für 4, 24, 48 und 72 Stunden mit (+) und ohne (-) Östrogen (E) behandelt wurden. Die Raf-Mutante BXB ist kleiner als das endogen von den Zellen produzierte Raf-1. In den untransfizierten Ausgangszellen (ER/EB2-5) wird durch den monoklonalen Antikörper, der gegen Raf-1 gerichtet ist, nur das Raf-1 Protein detektiert. In BC415-1 und -2 wird ein zusätzliches Protein angefärbt, das in seiner Größe der Mutante BXB entspricht. Durch Zugabe von Tc zum Zellkulturmedium wird die Expression dieses Proteins nach 48 Stunden vollständig abgeschaltet. Der Entzug von Östrogen, der in diesen Zellen zu einem Proliferationsstop führt (Kempkes et al., 1995), beeinflusst weder die Expression von BXB noch von endogenem Raf-1.
- Abb. 9:: Luziferaseaktivität in verschiedenen Sublinien (BC389-2 bis -10) von stabil mit MS2a transfizierten Raji Zellen vor und nach Behandlung mit Doxyciclin (DC).
- Abb. 10:: Vektor BC89
- Abb. 11:: Vektor PUHD15-1
- Abb. 12:: Vektor PUHD15-20
- Abb. 13:: Vektor PHEBOPL
- Abb. 14:: Vektor BC90
- Abb. 15:: Vektor PUHC13-3
- Abb. 16:: Vektor Ga38-14
- Abb. 17:: Vektor BC250
- Abb. 18:: Vektor BC315A
- Abb. 19:: Vektor BC311
- Abb. 20:: Vektor CR276

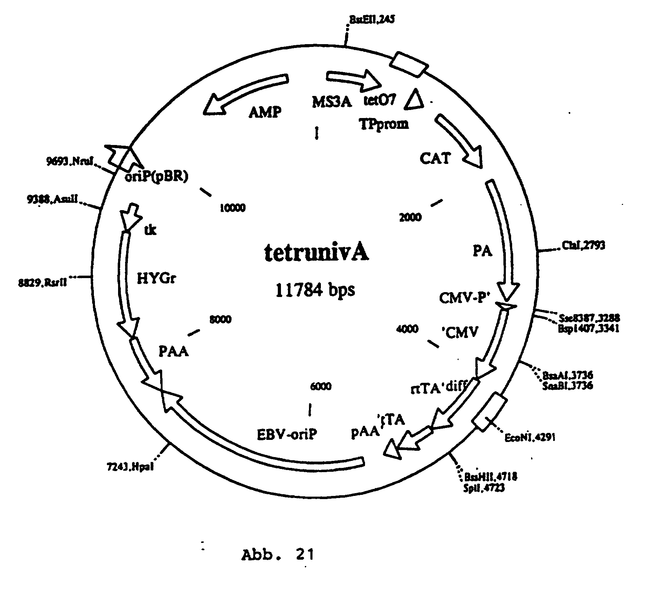

- Abb. 21:: Vektor tetrunivA
- Abb. 22:: Vektor BC252

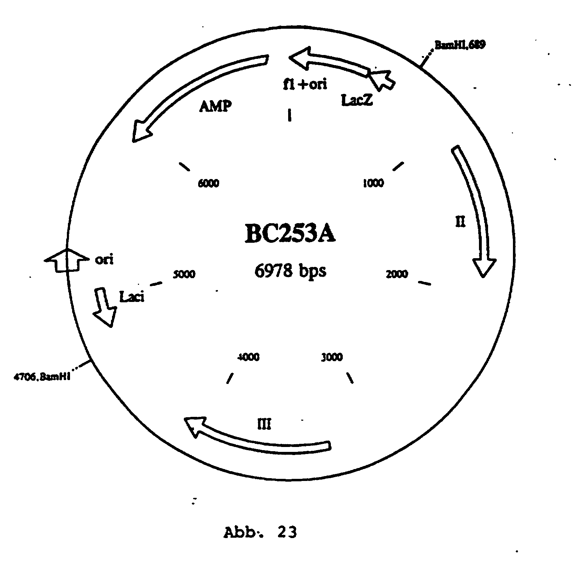

- Abb. 23:: Vektor BC253A
- Abb. 24:: Vektor BC266A
- Abb. 25:: Vektor BC252rev
- Abb. 26:: Vektor PUHD172-1
- Abb. 27:: Vektor BC364
- Abb. 28:: Vektor BC392

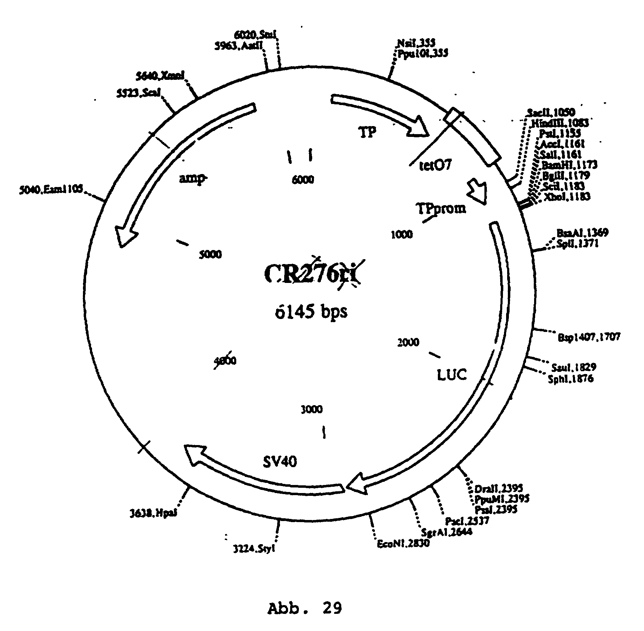

- Abb. 29:: Vektor CR276ri

### Literatur

Bouvier, G., Hergenhahn, M., Polack, A., Bornkamm, G.W., and Bartsch, H. (1993). Validation of two tes systems for detecting tumour promoters and EBV inducers: comparative responses of several agents in DR-CA1 Raji cells and in human granulocytes. Carcinogenesis 14, 1573-1578.
Bruder, J.T., Heidecker & U.R. Rapp, Serum-, TPA-, and Rasinduced expression from Ap-1/Ets-driven promoters requires Raf-1 kinase. Genes Dev. 6: 545-56 (1992).
Gazin, C., Dinechin, S.D., Hampe, A., Masson, J.M., Martin, P., Stehelin, D., and Galibert. F. (1984). Nucleotide sequence of the human c-myc locus: provocative open reading e within the first exon. EMBO J. *3*, 383-387.
Gossen, M., Freundlieb, S., Bender, G., Müller, G., Hillen, W., and Bujard, H. (1995). Transcriptional activation by tetracyclines in mammalian cells. Science *268*, 1766-1769.
Gossen, M. and Bujard, H. (1992). Tight control of gene expression in mammalian cells by tetracyclineresponsive promoters. Proc. Natl. Acad. Sci. U.S. A. *89*, 5547-5551.
Hammerschmidt, W. and Sugden, B. (1988). Identification and characterization of oriLyt, a lytic origin of DNA replication of Epstein-Barr virus. Cell 55, 427-433.
Hammerschmidt, W. and Sugden, B. (1989). Genetic analysis of immortalizing functions of Epstein-Barr virus in human B lymphocytes. Nature 340, 393-397.
Kempkes, B., Spitkovsky, D., Jansen-Dürr, P., Ellwart, J.W., Kremmer, E., Delecluse, H.-J., Rottenberger, C., Bornkamm, G.W., and Hammerschmidt, W. (1995). B-cell proliferation and induction of early G₁-regulating proteins by Epstein-Barr virus mutants conditional for EBNA2. EMBO J. *14*, 88-96.
Meitinger, C., Strobl, L.J., Marschall, G., Bornkamm, G.W., and Zimber-Strobl, U. (1994). Crucial sequences within the Epstein-Barr virus TP1 promoter for EBNA2-mediated transactivation and interaction of EBNA2 with its responsive element. J. Virol. *68*, 7497-7506.
Middleton, T. and Sugden, B. (1994). Retention of plasmid DNA in mammalian cells is enhanced by binding of the Epstein-Barr virus replication protein EBNA1. J. Virol. *68*, 4067-4071.
Polack, A., Strobl, L., Feederle, R., Schweizer, M., Koch, E., Eick, D., Wiegand, H., and Bornkamm, G.W. (1991). The intron enhancer of the immunoglobulin kappa gene activates c-myc but does not induce the Burkitt specific promoter shift. Oncogene *6*, 2033-2040.
Polack, A., Laux, G., Hergenhahn, M., Kloz, U., Rösner, H., Hecker, E., and Bornkamm, G.W. (1992). Shortterm assays for detection of conditional cancerogens L Construction of DR-CAT Raji cells and some of their characteristics as tester cells. Int. J. Cancer *50*, 611-616.
Sugden, B., Marsh, K., and Yates, J. (1985). A vector that replicates as a plasmid and can be efficiently selected in B-lymphoblasts transformed by Epstein-Barr virus. Mol. Cell Biol. *5*, 410-413.
Xu. L., Yee, J.K., Wolff, J.A., and Friedmann, T. (1989). Factors affecting long-term stability of Moloney murine leukemia virus-based vectors. Virology *171*, 331-341.
Yates, J., Warren, N., Reisman, D., and Sugden, B. (1984). A cis-acting element from the Epstein-Barr viral genome that permits stable replication of recombinant plasmids in latently infected cells. Proc. Natl. Acad. Sci. U. S. A. *81*, 3806-3810.
Yates, J.L., Warren, N., and Sugden, B. (1985). Stable replication of plasmids derived from Epstein-Barr virus in various mammalian cells. Nature *313*, 812-815.
Zimber Strobl, U., Kremmer, E, Grasser, F., Marschall, G., Laux, G., and Bornkamm, G.W. (1993). The Epstein-Barr virus nuclear antigen 2 interacts with an EBNA2 responsive cis-element of the terminal protein 1 gene promoter. EMBO J. *12*, 167-175.
Zimber Strobl, U., Strobl, L.J., Meitinger, C., Hinrichs, R., Sakai, T., Furukawa, T., Honjo, T., and Bornkamm, G.W. (1994). Epstein-Barr virus nuclear antigen 2 exerts its transactivating function through interaction with recombination signal binding protein RBP-J kappa, the homologue of Drosophila Suppressor of Hairless. EMBO J. *13*, 4973-4982.
Zimber-Strobl, U., Suentzenich, K.-O., Laux, G., Eick, D., Cordier, M., Calender, A., Billaud, M., Lenoir, G.M., and Bornkamm, G.W. (1991). Epstein-Barr virus nuclear antigen 2 activates transcription of the terminal protein gene. J. Virol. *65*, 415-

## Patentansprüche

1. In Eukaryontenzellen episomal replizierender, nicht integrierender Vektor zur konditionalen Genexpression in Eukaryonten, enthaltend, in funktioneller Verbindung, auf einem DNA-Molekül , zumindest eine Polynukleotidsequenz, die für ein mit Tetrazyklin oder seinen Derivaten oder Analogen regulierbares Transaktivator-fusionsprotein (tTA) kodiert, wobei tTA einen prokaryontischen Tet-Repressor und einen Transkriptionsaktivator, umfaßt einen tTA-responsiblen Promotor, der die tet-Operator-Sequenz (tetO) enthält, an die tTA binden kann, eine Polynukleotidsequenz, die für mindestens ein Gen von Interesse (GOI) kodiert, eine Polyadenylierungsstelle (PAA), eine Polynukleotidsequenz für zumindest einen selektierbaren Marker und Polynukleotidsequenzen, die eine episomale Replikation des Vektors in Eukaryonten bewirken.

2. Vektor nach Anspruch 1
**dadurch gekennzeichnet,**
**daß** der Tet-Repressor von tTA ein von Tn10-stammender Tet-Repressor ist.

3. Vektorem nach Ansprüch 1 oder 2,
**dadurch gekennzeichnet,**
**daß** der Transkriptionsaktivator aus dem Herpes simplex-Virus-Virionprotein 16 stammt oder aus mindestens einem Element der Gruppe saurer, prolinreicher, serin/threonin-reicher und glutaminreicher Transkriptionsaktivatorpolypeptide ausgewählt ist, oder eine Interaktionsdomäne ist, ausgewählt aus mindestens einem Element der Gruppe, bestehend aus einer Leucin-Zipper-Domäne, einer Helix-Loop-Helix-Domäne und einer Zinkfinger-Domäne, oder
eine Interaktionsdomäne aus einem TATA-Bindungsprotein ist.

4. Vektor nach einem oder mehreren der vorhergehenden Ansprüche,
**dadurch gekennzeichnet,**
**daß** die für tTA kodierende Polynukleotidsequenz unter Kontrolle eines konstitutiv aktiven Promotors steht, bevorzugt eines CMV-, tk- oder c-myc-Promotors oder Teilen hiervon, oder die tTA-kodierende Polynukleotidsequenz unter Kontrolle eines tTA-responsiblen Promotors steht.

5. Vektor nach einem oder mehreren der vorhergehenden Ansprüche,
**dadurch gekennzeichnet,**
**daß** das Gen von Interesse für ein humanes oder ein virales oder ein bakterielles Protein kodiert, bevorzugt ausgewählt aus mindestens einem Element der Gruppe, bestehend aus Transkriptionsfaktoren, Onkogenen, Signalübertragungsmolekülen, Enzymen, Cytokinen, Zelladhäsionsproteinen, Tumorantigenen, co-stimulatorischen Signalmolekülen und ein nicht mit dem Empfänger identisches HLA.

6. Vektor nach einem oder mehreren der vorhergehenden Ansprüche,
**dadurch gekennzeichnet,**
**daß** der tTA-responsible Promotor einen Minimalpromotor enthält oder
**daß** der tTA-responsible Promotor zumindest Teile des TP-Promotors des EBV, des CMV-, tk- oder c-myc-Promotors enthält oder
**daß** der tTA-responsible Promotor den TP-Promotor von EBV enthält, bei dem das EBNA2-responsible Element durch die tet-Operator-Sequenz (tetO) ersetzt oder ergänzt wurde, oder
**daß** der tTA-responsible Promotor ein Minimalpromotor in funktioneller Verbindung mit tetO ist, oder
**daß** der tTA-responsible Promotor ein CMV oder tk-Minimalpromotor oder ein TP-Minimalpromotor des EBV, je in funktioneller Verbindung mit tetO, ist, oder
**daß** der tTA-responsible Promotor ein c-myc-P1-Promotor in funktioneller Verbindung mit tetO ist.

7. Vektor nach einem oder mehreren der vorhergehenden Ansprüche,
**dadurch gekennzeichnet,**
**daß** die tet-Operator-Sequenz (tetO) in multipler Kopienanzahl vorliegt, bevorzugt in sieben Kopien.

8. Vektor nach einem oder mehreren der vorhergehenden Ansprüche,
**dadurch gekennzeichnet,**
**daß** die Polynukleotidsequenz, die für einen selektierbaren Marker kodiert, das tk-Gen oder das Neomycin-Resistenzgen oder das Hygromycin-Resistenzgen ist.

9. Vektor nach einem oder mehreren der vorhergehenden Ansprüche,
**dadurch gekennzeichnet,**
**daß** die Polynukleotidsequenzen, die eine episomale Replikation des Vektors in Eukaryonten bewirken, den Replikationsursprung (ori) von EBV (oriP) oder von BPV enthalten, oder
**daß** die Polynukleotidsequenz, die eine episomale Replikation des Vektors in Eukaryonten bewirkt, für EBNA1 kodiert.

10. Vektor nach einem oder mehreren der vorhergehenden Ansprüche,
**dadurch gekennzeichnet,**
**daß** der Vektor die Sequenzen für den lytischen Origin des EBV und die terminale Repeat-Struktur als Verpackungssequenz (TR) enthält, oder
**daß** der Vektor Sequenzen für die Replikation in Bakterienzellen enthält.

11. Vektor nach einem oder mehreren der vorhergehenden Ansprüche,
**dadurch gekennzeichnet,**
**daß** tetO im tTA-responsiblen Promotor durch Bindung des Tet-Repressors positiv oder negativ regulierbar ist.

12. Prokaryontenzelle oder Eukaryontenzelle, ausgenommen Keimbahnzellen, enthaltend einen Vektor nach einem oder mehreren der vorhergehenden Ansprüche.

13. B-Zelle, die EBV-positiv sein kann, enthaltend einem Vektor nach einem oder mehreren des Ansprüche 1-11.

14. EBV-positive Eukaryontenzelle, ausgenommen Keimbahnzellen, oder Prokaryontenzelle, enthaltend einen Vektor nach einem oder mehreren des Ansprüchs 1-11.

15. Arzneimittel, enthaltend einen Vektor nach einem oder mehreren des Ansprüche 1-11.

16. Arzneimittel, enthaltend eine Prokaryontenzelle oder Eukaryontenzelle nach Anspruch 12, 13 oder 14.

17. Verfahren zur konditionalen Expression eines oder mehrerer Gene von Interesse in vitro mit den nachfolgenden Schritten:
(a) Transfektion eines Vektors nach einen oder mehreren Ansprüche 1-11 in Eukaryontenzellen in stabiler oder transienter Form;
(b) Behandlung der Zellen mit Tetracyclin oder seinen Derivaten oder Analogen in einer solchen Konzentration, die die gewünschte Expression des Gens von Interesse ermöglicht; wahlweise
(c) Isolieren des produzierten Proteins aus der Zelle.

18. Verfahren zur konditionalen Expression eines oder mehrerer Gene von Interesse in vitro mit den nachfolgenden Schritten:
(a) Transfektion eines Vektors nach Anspruch 10 in eine EBV-positive Zelle;
(b) Induktion des lytischen Zyklus;
(c) Ernten des Überstandes;
(d) wahlweise Konzentrieren des Überstandes;
(e) Infektion von EBV-Rezeptoren tragenden Zellen;
(f) Behandlung der Zellen mit Tetracyclin oder seinen Derivaten oder Analogen in einer solchen Konzentration, die die gewünschte Expression des Gens von Interesse ermöglicht; wahlweise
(g) Isolieren des produzierten Proteins aus der Zelle.

19. Verfahren nach Anspruch 18,
**dadurch gekennzeichnet,**
**daß** als Zellen B-Zellen oder von B-Zellen abgeleitete Tumorzellen verwendet werden.

20. EBV-Viruspartikel, enthaltend einen oder mehrere der Vektoren nach einem oder mehreren der vorhergehenden Ansprüche.

## Claims

1. Vector being non-integrating and episomally replicating in eukaryotic cells for the conditional expression of genes in eukaryotes, comprising, operably linked on one DNA molecule, at least one polynucleotide sequence coding for a transactivator fusion protein (tTA) which can be controlled by tetracycline or its derivatives or analogues, the tTA comprising a prokaryotic tet repressor and an activator of transcription, a promoter responsive to tTA containing the tet operator sequence (tetO) to which tTA is able to bind, a polynucleotide sequence coding for at least one gene of interest (GOI), a polyadenylation site (PAA), a polynucleotide sequence for at least one selectable marker, and polynucleotide sequences causing episomal replication of the vector at least in eukaryotes.

2. Vector according to claim 1
**characterized in that**
said tet repressor of tTA a Tn10-derived tet repressor.

3. Vector according to claim 1 or 2
**characterized in that**
said tTA activator of transcription is derived from Herpes simplex virion protein 16, or is selected of at least one element of the group of acidic, proline-rich, serine/threonine-rich, and glutamine-rich transcriptional activator polypeptides, or is an interaction domain selected of at least one element of the group consisting of a leucin-zipper domain, a helix-loop-helix domain, and a zink finger domain, or is an interaction domain of a TATA binding protein.

4. Vector according to one or more of the preceding claims,
**characterized in that**
said polynucleotide sequence coding for tTA is under the control of a constitutively active promoter, preferably a CMV, tk, or c-myc promoter or portions thereof, or the polynucleotide sequence coding for tTA is under the control of a promoter responsive to tTA

5. Vector according to one or more of the preceding claims,
**characterized in that**
said gene of interest is a gene coding for a human or a viral or a bacterial protein, preferably selected from at least one element of the group consisting of transcription factors, oncogenes, signal transducing molecules, enzymes, cytokins, cellular adhesion proteins, tumour antigens, co-stimulatory signalling molecules, and a HLA not identical to that of the recipient.

6. Vector system according to one or more of the preceding claims,
**characterized in that**
said tTA-responsive promoter contains a minimal promoter, or said tTA-responsive promoter contains at least portions of the TP promoter of EBV, the CMV, tk, or the c-myc promoter, or said tTA-responsive promoter contains the TP promoter of EBV wherein the tet operator sequence (tetO) is substituted for or added to the EBNA2-responsive element, or said tTA-responsive promoter is a minimal promoter operably linked to tetO, or said tTA-responsive promoter is a CMV or tk minimal promoter or a TP minimal promoter of EBV, each operably linked to tetO, or said tTA-responsive promoter is a c-myc P1 promoter operably linked to tetO.

7. Vector according to one or more of the preceding claims,
**characterized in that**
said tet operator sequence (tetO) is present in multiple copy number, and preferably in seven copies.

8. Vector according to one or more of the preceding claims,
**characterized in that**
said polynucleotide sequence coding for a selectable marker is the tk gene or the neomycin resistance gene or the hygromycin resistance gene.

9. Vector according to one or more of the preceding claims,
**characterized in that**
said polynucleotide sequence causing the episomal replication of the vector in eukaryotes contains the origin of replication (ori) of EBV (oriP) or BPV, or said polynucleotide sequence causing the episomal replication of the vector in eukaryotes is a polynucleotide sequence coding for EBNA1.

10. Vector according to one or more of the preceding claims,
**characterized in that**
said vector contains the sequence of the lytic origin of EBV, and the terminal repeat structure functioning as packaging sequence (TR), or said vector contains sequences allowing its replication in bacterial cells.

11. Vector according to one or more of the preceding claims,
**characterized in that**
said tetO in the promoter responsive to tTA can be controlled in a positive or negative manner by binding of the tet repressor.

12. Eukaryotic cell or prokaryotic cell, with the proviso of germ line cells, containing a vector according to one or more of the preceding claims,

13. B cell which may be EBV-positive, containing a vector in accordance with one or more of claims 1-11.

14. Eukaryotic cell being EBV-positive, with the proviso of germ line cells, or a prokaryotic cell, containing a vector in accordance with one or more of claims 1-11.

15. Pharmaceutical composition, containing a vector in accordance with one or more of claims 1-11.

16. Pharmaceutical composition, containing a prokaryotic or eukaryotic cell in accordance with claim 12, 13 or 14.

17. Process for the conditional expression of one or more genes of interest in vitro comprising the following steps of:
(a) transfection of a vector according to one or more of claims 1-11 into eukaryotic cells in stable or transient form;
(b) treatment of the cells with tetracycline or its derivatives or analogues in a concentration sufficient to enable the desired expression of the gene of interest; and optionally
(c) isolating the protein produced from the cell.

18. Process for the conditional expression of one or more genes of interest in vitro comprising the following steps of:
(a) transfection of a vector according to claim 10 into an EBV-positive cell;
(b) induction of the lytic cycle;
(c) harvesting the supernatant;
(d) optionally concentrating the supernatant;
(e) infection of cells bearing EBV-receptors;
(f) treatment of cells with tetracyline or its derivatives or analogues in a concentration sufficient to enable the desired expression of the gene of interest; and optionally
(g) isolating the protein produced from the cell.

19. Process according to claim 18
**characterized in that**
B cells or tumour cells derived from B cells are used as said cells.

20. EBV viral particles containing one or more vectors according to one or more of the preceding claims.

## Revendications

1. Vecteur non intégrant à réplication épisomique dans des cellules d'eucaryotes pour l'expression conditionnelle d'un gène dans des eucaryotes, contenant, en liaison fonctionnelle, sur une molécule d'ADN, au moins une séquence polynucléotidique, qui code pour une protéine de fusion de transactivateur (tTA) régulable avec de la tétracycline ou ses dérivés ou analogues, le tTA comprenant un répresseur Tet procaryote et un activateur de transcription, un promoteur sensible au tTA, qui contient la séquence tet-opérateur (tetO), à laquelle le tTA peut se lier, une séquence polynucléotidique, qui code pour au moins un gène d'intérêt (GDI), une position de polyadénylation (PAA), une séquence polynucléotidique pour au moins un marqueur sélectionnable et des séquences polynucléotidiques qui effectuent une réplication épisomique du vecteur dans des eucaryotes.

2. Vecteur selon la revendication 1, **caractérisé en ce que** le répresseur Tet du tTA est un répresseur Tet provenant du Tn10.

3. Vecteur selon la revendication 1 ou 2, **caractérisé en ce que** l'activateur de transcription provient de la protéine de virion du virus Herpès simplex 16 ou est choisi parmi au moins un élément du groupe des polypeptides d'activateurs de transcription acides, riches en proline, riches en sérine/thréonine et riches en glutamine ou est un domaine d'interaction choisi parmi au moins un élément du groupe constitué d'un domaine leucine-zipper, d'un domaine hélice-boucle-hélice et d'un domaine de doigts de zinc, ou est un domaine d'interaction d'une protéine de liaison à TATA.

4. Vecteur selon une ou plusieurs des revendications précédentes, **caractérisé en ce que** la séquence polynucléotidique codant pour tTA est sous le contrôle d'un promoteur actif de manière constitutive, de préférence un promoteur CMV, tk ou c-myc ou de parties de ceux-ci, ou la séquence polynucléotidique codant pour tTA est sous le contrôle d'un promoteur sensible au tTA.

5. Vecteur selon une ou plusieurs des revendications précédentes, **caractérisé en ce que** le gène d'intérêt pour une protéine humaine ou une protéine virale ou une protéine bactérienne, de préférence choisie parmi au moins un élément du groupe constitué des facteurs de transcription, des oncogènes, des molécules de transfert de signal, des enzymes, des cytokines, des protéines d'adhérence de cellules, des antigènes tumoraux, des molécules signal costimulatrices et d'un HLA qui n'est pas identique au récepteur.

6. Vecteur selon une ou plusieurs des revendications précédentes, **caractérisé en ce que** le promoteur sensible au tTA contient un promoteur minimal, ou
**en ce que** le promoteur sensible au tTA contient au moins des parties du promoteur TP de l'EBV et du promoteur CMV, tk ou c-myc, ou
**en ce que** le promoteur sensible au tTA contient le promoteur TP de l'EBV, l'élément sensible à l'EBNA2 étant remplacé ou complété par la séquence tet-opérateur (tetO), ou
**en ce que** le promoteur sensible au tTA est un promoteur minimal en liaison fonctionnelle avec tetO, ou
**en ce que** le promoteur sensible au tTA est un promoteur CMV ou un promoteur minimal tk ou encore un promoteur minimal TP de l'EBV, respectivement en liaison fonctionnelle avec tetO, ou
**en ce que** le promoteur sensible au tTA est un promoteur c-myc-P1 en liaison fonctionnelle avec tetO.

7. Vecteur selon une ou plusieurs des revendications précédentes, **caractérisé en ce que** la séquence tet-opérateur (tetO) est présente en nombre multiple de copies, de préférence en sept copies.

8. Vecteur selon une ou plusieurs des revendications précédentes, **caractérisé en ce que** la séquence polynucléotidique, qui code pour un marqueur sélectionnable, est le gène tk ou le gène de résistance à la néomycine ou le gène de résistance à l'hygromycine.

9. Vecteur selon une ou plusieurs des revendications précédentes, **caractérisé en ce que** les séquences polynucléotidiques, qui effectuent une réplication épisosomique du vecteur dans des eucaryotes, contiennent la source de réplication (ori) de l'EBV (oriP) ou du BPV, ou
**en ce que** la séquence de polynucléotides, qui effectue une réplication épisomatique du vecteur dans des eucaryotes, code pour l'EBNA1.

10. Vecteur selon une ou plusieurs des revendications précédentes, **caractérisé en ce que** le vecteur contient les séquences pour l'origine lytique de l'EBV et la structure de répétition terminale comme séquence de conditionnement (TR), ou
**en ce que** le vecteur contient des séquences pour la réplication dans des cellules bactériennes.

11. Vecteur selon une ou plusieurs des revendications précédentes, **caractérisé en ce que** le tetO dans le promoteur sensible au tTA peut être régulé de manière positive ou négative par liaison du répresseur Tet.

12. Cellule procaryote ou cellule eucaryote, à l'exception des cellules des voies d'ensemencement, contenant un vecteur selon l'une ou plusieurs des revendications précédentes.

13. Cellule B, qui peut être EBV-positive, contenant un vecteur selon une ou plusieurs des revendications 1 à 11.

14. Cellule eucaryote EBV-positive, à l'exception des cellules des voies d'ensemencement, ou cellule procariote, contenant un vecteur selon une ou plusieurs des revendications 1 à 11.

15. Médicament contenant un vecteur selon une ou plusieurs des revendications 1 à 11.

16. Médicament contenant une cellule procariote ou une cellule eucaryote selon la revendication 12, 13 ou 14.

17. Procédé d'expression conditionnelle d'un ou plusieurs gènes d'intérêt in vitro avec les étapes suivantes
a) transfection d'un vecteur selon une ou plusieurs des revendications 1 à 11 dans des cellules eucaryotes sous forme stable ou transitoire;
b) traitement des cellules avec de la tétracycline ou ses dérivés ou analogues dans une concentration telle qu'elle permette l'expression souhaitée du gène d'intérêt ; éventuellement
c) isolement de la protéine produite à partir de la cellule.

18. Procédé pour l'expression conditionnelle d'un ou plusieurs gènes d'intérêt in vitro avec les étapes suivantes :
a) transfection d'un vecteur selon la revendication 10 dans une cellule EBV-positive;
b) induction du cycle lytique;
c) collecte du liquide surnageant;
d) éventuellement concentration du liquide surnageant;
e) infection de cellules portant des récepteurs EBV;
f) traitement des cellules avec de la tétracycline ou ses dérivés ou analogues, qui permet l'expression souhaitée du gène d'intérêt ; éventuellement
g) isolement de la protéine produite de la cellule.

19. Procédé selon la revendication 18, **caractérisé en ce que** l'on utilise comme cellules des cellules B ou des cellules tumorales issues de cellules B.

20. Particule de virus EBV contenant un ou plusieurs vecteurs selon une ou plusieurs des revendications précédentes.
